# EUROPEAN PATENT APPLICATION

(11) **EP 4 650 496 A1**
(43) Date of publication of application: **19.11.2025**
(21) Application number: 24219315.9
(22) Date of filing: 12.12.2024
(51) Int. Cl.: C30B 25/16, C30B 29/16

(54) **PREDICTION METHOD AND DEVICE FOR EPITAXIAL LAYER OF BETA-GA2O3 GROWN BY MOCVD**

(30) Priority: 15.05.2024 CN 202410603557
(71) Applicant: Hangzhou Fujia Gallium Technology Co. Ltd., Hangzhou Zhejiang 311421 (CN)
(72) Inventor: QI, Hongji, Hangzhou, Zhejiang, 311421 (CN); CHEN, Duanyang, Hangzhou, Zhejiang, 311421 (CN); YANG, Zhenni, Hangzhou, Zhejiang, 311421 (CN); TIAN, Ka, Hangzhou, Zhejiang, 311421 (CN)
(74) Representative: KIPA AB

(57) **Abstract**

A prediction method and a device for epitaxial layer of beta-GazOs grown by MOCVD are provided. The method includes acquiring preset growth parameters, and inputting the preset growth parameters into a trained epitaxial layer prediction model, the preset growth parameters includes an O/Ga ratio, chamber pressure, temperature, and growth time; outputting predicted data of the beta-GazOs epitaxial layer corresponding to the preset growth parameters by the epitaxial layer prediction model, the predicted data at least includes full width at half maximum difference. In the present disclosure, In the present disclosure, the machine learning model is used to predict the epitaxial layer, and the preset growth parameters are adjusted according to the predicted data to select the growth parameters suitable for the process window of beta-GazOs epitaxial growth, and then the generated parameters are input into MOCVD for epitaxy. By combining machine learning with MOCVD process, high-quality two-dimensional step-flow growth with TMGa and O₂ as reactants on unintentional oblique substrate is realized, and the quality of beta-Ga₂O₃ epitaxial layer grown by MOCVD is ensured.

## Description

### CROSS-REFERENCE TO RELATED APPLICATIONS

This application claims priority to Chinese Patent Application No. 202410603557.8, filed on May 15, 2024, the content of all of which is incorporated herein by reference.

### FIELD OF THE DISCLOSURE

The present disclosure relates to the technical field of beta-Ga₂O₃ epitaxial layer, in particular to a prediction method and a device for epitaxial layer of beta-Ga₂O₃ grown by MOCVD.

### BACKGROUND

At present, when beta-Ga₂O₃ is epitaxially grown by Metal-organic Chemical Vapor Deposition (MOCVD), the reaction temperature of TMGa is higher, and the oxidation of O₂ is stronger, which leads to the pre-reaction in high temperature environment, that is, the reactants react to generate beta-Ga₂O₃ before reaching the substrate. The powder produced by the pre-reaction serves as a new nucleation point on the surface of the substrate, which promotes the three-dimensional growth of the epitaxial layer, thus seriously deteriorating the quality of the epitaxial layer.

Therefore, the existing technology still needs to be improved and developed.

### BRIEF SUMMARY OF THE DISCLOSURE

To overcome the defects of the background, the present disclosure provides a prediction method and a device for epitaxial layer of beta-Ga₂O₃ grown by Metal-organic Chemical Vapor Deposition (MOCVD) to effectively solve the problems involved in the prior art.

To solve the problems, in a first aspect, the present disclosure provides a prediction method for epitaxial layer of beta-Ga₂O₃ grown by MOCVD, including:
acquiring preset growth parameters, and inputting the preset growth parameters into a trained epitaxial layer prediction model, the preset growth parameters include an O/Ga ratio, chamber pressure, temperature, and growth time;
outputting predicted data of the beta-Ga₂O₃ epitaxial layer corresponding to the preset growth parameters by the epitaxial layer prediction model, the predicted data at least includes full width at half maximum difference.

The training process of the epitaxial layer prediction model includes:
acquiring a training dataset, the training dataset includes a plurality of training data groups, and each training data group includes training growth parameters and label data of a beta-Ga₂O₃ epitaxial layer;
inputting the training growth parameters in the training dataset into a preset machine learning model, and outputting the training predicted data of the beta-Ga₂O₃ epitaxial layer by the preset machine learning model;
training the preset machine learning model based on the training predicted data and the label data to obtain the trained epitaxial layer prediction model.

The step of training the preset machine learning model based on the training predicted data and the label data to obtain the trained epitaxial layer prediction model includes:
calculating a predicted difference value between the training predicted data and the label data;
when the predicted difference value is less than or equal to a preset difference threshold, determining a loss function term based on the training predicted data and the label data, and training the preset machine learning model based on the loss function term;
when the predicted difference value is greater than the preset difference threshold, determining the loss function term based on the training predicted data and the label data, setting a penalty term for the training predicted data, and training the preset machine learning model based on the loss function term and the penalty term.

Both of the training predicted data and the label data include full width at half maximum difference; the step of calculating a predicted difference value between the training predicted data and the label data includes:
reading a predicted full width at half maximum difference in the training predicted data and a labeled full width at half maximum difference in the label data, the predicted full width at half maximum difference and the labeled full width at half maximum difference are both used for reflecting a difference between the full width at half maximum after epitaxy and the full width at half maximum before epitaxy;
calculating a difference between the predicted full width at half maximum difference and the labeled full width at half maximum difference to obtain the predicted difference value.

The predicted data further includes thickness and/or surface roughness.

After the step of outputting predicted data of the beta-Ga₂O₃ epitaxial layer corresponding to the preset growth parameters by the epitaxial layer prediction model, the method further includes:
detecting whether the predicted data meets preset requirements;
when the predicted data does not meet the preset requirements, modifying the preset growth parameters to obtain modified growth parameters;
taking the modified growth parameters as the preset growth parameters, and re-executing the step of inputting the preset growth parameters into the trained epitaxial layer prediction model until the predicted data meets the preset requirements;
taking the preset growth parameters corresponding to the predicted data meeting the preset requirements as target growth parameters, and inputting the target growth parameters into MOCVD for beta-Ga₂O₃ epitaxy.

The prediction method for epitaxial layer of beta-Ga₂O₃ grown by MOCVD further includes:
generating a fine-tuning dataset based on the target growth parameters and corresponding epitaxial data;
fine-tuning the epitaxial layer prediction model based on the fine-tuning dataset.

In a second aspect, the present disclosure further provides a prediction device for epitaxial layer of beta-Ga₂O₃ grown by MOCVD, including:
an acquisition module, used for acquiring preset growth parameters, and inputting the preset growth parameters into a trained epitaxial layer prediction model, the preset growth parameters include an O/Ga ratio, chamber pressure, temperature, and growth time;
a prediction module, used for outputting predicted data of the beta-Ga₂O₃ epitaxial layer corresponding to the preset growth parameters by the epitaxial layer prediction model, the predicted data at least includes full width at half maximum difference.

In a third aspect, the present disclosure further provides a computer-readable storage medium, one or more programs are stored in the computer-readable storage medium, and the one or more programs can be executed by one or more processors to realize the steps in the prediction method.

In a fourth aspect, the present disclosure further provides a terminal device, including a processor and a memory;
a computer-readable program executable by the processor is stored in the memory;
when the processor executes the computer-readable program, the processor realizes any one of the steps in the prediction method for epitaxial layer of beta-Ga₂O₃ grown by MOCVD.

Compared with the prior art, the present disclosure at least has following beneficial effects: a prediction method and a device for epitaxial layer of beta-Ga₂O₃ grown by MOCVD are provided. The method includes acquiring preset growth parameters, and inputting the preset growth parameters into a trained epitaxial layer prediction model, the preset growth parameters includes an O/Ga ratio, chamber pressure, temperature, and growth time; outputting predicted data of the beta-Ga₂O₃ epitaxial layer corresponding to the preset growth parameters by the epitaxial layer prediction model, the predicted data at least includes full width at half maximum difference. In the present disclosure, the machine learning model is used to predict the epitaxial layer, and the preset growth parameters are adjusted according to the predicted data to select the growth parameters suitable for the process window of beta-Ga₂O₃ epitaxial growth, and then the generated parameters are input into MOCVD for epitaxy to improve the quality of the beta-Ga₂O₃ epitaxial layer grown by MOCVD.

### BRIEF DESCRIPTION OF THE DRAWINGS

To more clearly describe the technical schemes in the embodiments of the present disclosure, the attached drawings used in the embodiments are briefly described herein. Obviously, the attached drawings in the following description are only some examples of the present disclosure. For those skilled in the art, other drawings can be obtained according to the following drawings without inventive labor.
Fig. 1 is a flow chat of a prediction method for epitaxial layer of beta-Ga₂O₃ grown by MOCVD provided in one embodiment of the present disclosure.
Fig. 2 is a principle flow chat of the prediction method for epitaxial layer of beta-Ga₂O₃ grown by MOCVD provided in an implementation of the present disclosure.
Fig. 3 is a schematic diagram of a determination coefficient of a thickness of epitaxial layer of the epitaxial layer prediction model trained on a training dataset.
Fig. 4 is a schematic diagram of a determination coefficient of a full width at half maximum difference of the epitaxial layer prediction model trained on the training dataset.
Fig. 5 is a schematic diagram of a determination coefficient of a thickness of epitaxial layer of the epitaxial layer prediction model on a test set.
Fig. 6 is a schematic diagram of a determination coefficient of a full width at half maximum difference of the epitaxial layer prediction model trained on the test set.
Fig. 7 is a comparison diagram of the training dataset and an expanded training dataset.
Fig. 8 is a structural schematic diagram of a prediction device for epitaxial layer of beta-Ga₂O₃ grown by MOCVD provided by one embodiment of the present disclosure.
Fig. 9 is a structural schematic diagram of a terminal device provided by one embodiment of the present disclosure.

### DETAILED DESCRIPTION OF EMBODIMENTS

The present disclosure provides a prediction method and a device for epitaxial layer of beta-Ga₂O₃ grown by Metal-organic Chemical Vapor Deposition (MOCVD). To make the purpose, technical schemes and effects of the present disclosure more clear and definite, the present disclosure is further described in detail with reference to the attached drawings and embodiments. It should be understood that the embodiments described herein are only used to explain the present disclosure, and are not used to limit the present disclosure.

It can be understood by those skilled in the art that the singular forms "a", "an", and "the" used herein can also include plural forms unless specifically stated. It should be further understood that the term "comprising" used in the specification of this application refers to the presence of said features, integers, steps, operations, elements and/or components, but does not exclude the presence or addition of one or more other features, integers, steps, operations, elements, components and/or groups thereof. It should be understood that when we say that an element is "connected" or "coupled" to another element, it can be directly connected or coupled to the other element, or intervening elements may also exist. Furthermore, "connected" or "coupled" as used herein may include wireless connection or wireless coupling. As used herein, the term "and/or" includes all or any unit and all combinations of one or more associated listed items.

It can be understood by those skilled in the art that unless otherwise defined, all terms (including technical terms and scientific terms) used herein have the same meaning as commonly understood by those skilled in the art to which the present disclosure belongs. It should also be understood that terms, such as those defined in general dictionaries, should be understood to have meanings consistent with those in the context of the prior art, and will not be interpreted in an idealized or overly formal sense unless they are specifically defined as here.

It should be understood that the sequence number and size of each step in the embodiment do not mean the order of execution, and the order of execution of each process is determined by its function and internal logic, and should not constitute any restrictions on the implementation process of this embodiment of the application.

At present, when beta-Ga₂O₃ is epitaxially grown by MOCVD, the reaction temperature of TMGa is higher, and the oxidation of O₂ is stronger, which leads to the pre-reaction in high temperature environment, that is, the reactants react to generate beta-Ga₂O₃ before reaching the substrate. The powder produced by the pre-reaction serves as a new nucleation point on the surface of the substrate, which promotes the three-dimensional growth of the epitaxial layer, thus seriously deteriorating the quality of the epitaxial layer.

To solve the problems, some researchers have proposed to use TEGa and O₂ with low reaction dimensions, but this scheme causes the problem of low lateral thermal diffusion rate of atoms on the substrate, and it is necessary to use the substrate chamfering to artificially create chamfering angles and shorten the width of steps. However, the actual temperature of each MOCVD epitaxial device is different, which requires oblique cutting of the substrate at different angles, which not only increases the labor cost and processing difficulty, but also is not universal. Some researchers suggests that N₂O with higher pyrolysis temperature can be used as the oxygen source, so that TMGa and N₂O are activated and reacted only when they are close to the substrate. However, the ultra-high temperature epitaxial growth of oxides is a difficult problem for MOCVD device, and there are many technical difficulties to be overcome in the service life of device and the uniformity of epitaxial layer.

Through research, the present disclosure founds the main problem that TMGa and O₂ can't achieve high-quality epitaxy as reactants is that at present, the formulation of MOCVD process is regulated by the traditional MOCVD process debugging relying on the subjective experience of process engineers, and this experience-oriented regulation method is prone to misjudgment leading to missing the best process window. Especially for the epitaxy of beta-Ga₂O₃, the real optimal epitaxial process window is very narrow, so it is necessary to eliminate human judgment errors as much as possible.

Therefore, in one embodiment of the present disclosure, preset growth parameters are obtained and input into a trained epitaxial layer prediction model. The preset growth parameters include an O/Ga ratio, chamber pressure, temperature, and growth time, and the predicted data of the beta-Ga₂O₃ epitaxial layer corresponding to the preset growth parameters is output by the epitaxial layer prediction model. In the present disclosure, the machine learning model is used to predict the epitaxial layer and the preset growth parameters are adjusted according to the predicted data to select the growth parameters suitable for the process window of beta-Ga₂O₃ epitaxial growth, and then the generated parameters is input into MOCVD for epitaxy. In this way, combining machine learning with MOCVD process, high-quality two-dimensional step-flow growth with TMGa and O₂ as reactants on unintentional oblique substrate is realized, and the quality of beta-Ga₂O₃ epitaxial layer grown by MOCVD is ensured.

The present disclosure is further explained by describing the embodiment with the attached drawings.

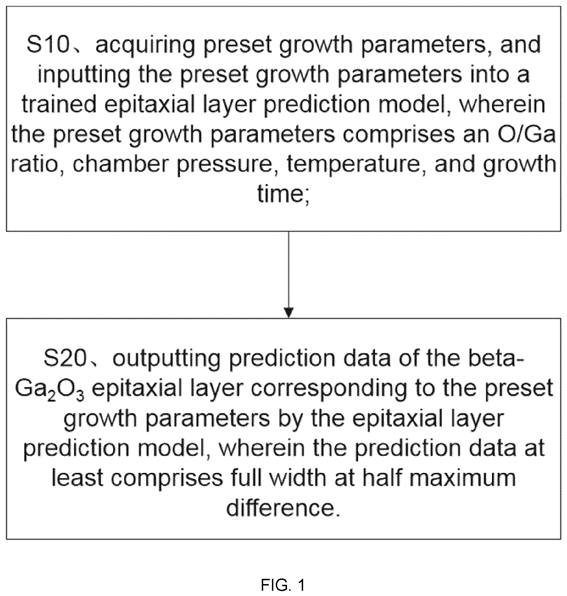

As shown in Fig. 1, the present disclosure provides a prediction method for epitaxial layer of beta-Ga₂O₃ grown by MOCVD, including:
S10, acquiring preset growth parameters, and inputting the preset growth parameters into a trained epitaxial layer prediction model;
S20, outputting predicted data of the beta-Ga₂O₃ epitaxial layer corresponding to the preset growth parameters by the epitaxial layer prediction model.

The preset growth parameters are the process parameters that affect the MOCVD epitaxial growth, which affects the diffusion coefficient *D^{m}* and deposition coefficient *Fⁿ* of MOCVD epitaxial growth, the diffusion coefficient *D^{m}* is used to describe the lateral diffusion rate of atoms on the substrate, and the deposition coefficient *Fⁿ* is used to describe the vertical deposition rate of atoms on the substrate. When *D^{m}*>*Fⁿ*, the diffusion rate of atoms on the substrate is higher than the deposition rate of epitaxial layers, and the atoms adsorbed on the substrate surface will not be directly combined into the crystal lattice. These atoms are in thermal motion on the substrate surface, and the diffusion coefficient *D^{m}* is positively correlated with the temperature. Meanwhile, the epitaxial layer shows 2-dimensional growth. When *D^{m}<Fⁿ,* the deposition rate is greater than the diffusion rate, and the epitaxial layer shows 3-dimensional island-shaped growth.

The process parameters that affect the diffusion coefficient *D^{m}* and deposition coefficient *Fⁿ* of MOCVD epitaxial growth can include an O/Ga ratio, chamber pressure, temperature, growth time and a flow rate of organic sources. Therefore, in the embodiment of the present disclosure, the preset growth parameters may include an O/Ga ratio, chamber pressure, temperature, and growth time. Of course, in practical application, the preset growth parameters may also include the flow rate of reactants. For instance, in one embodiment of the present disclosure, the reactants are TMGa and O₂, so the preset growth data also includes the flow rate of TMGa, and then the flow rate of O₂ can be determined according to the O/Ga ratio.

The epitaxial layer prediction model adopts machine learning model, such as a polynomial regression model, and so on. The epitaxial layer prediction model is used to predict the epitaxial data of the beta-Ga₂O₃ epitaxial layer, which is obtained by epitaxial growth on an unintentional oblique substrate by MOCVD epitaxial process with TMGa and O₂ as reactants. That is to say, the epitaxial layer prediction model can predict the epitaxial data of beta-Ga₂O₃ epitaxial layer obtained by MOCVD epitaxial growth of beta-Ga₂O₃ with TMGa and O₂ as the reaction under the preset growth parameters.

Further, when the epitaxial parameters are appropriate, the crystal quality of the epitaxial layer is expected to be better than the crystal quality of the substrate, and the FWHM of the epitaxial layer crystal is expected to be smaller than the FWHM of the substrate. Therefore, the predicted data can include a full width at half maximum difference (FWHM difference, ΔFWHM), and the quality of the epitaxial layer can be reflected by the FWHM difference. The FWHM difference is used to reflect a difference between the FWHM after epitaxy and the FWHM before epitaxy, that is, the FWHM difference is equal to the FWHM after epitaxy minus the FWHM before epitaxy. In addition, in practical application, in addition to the FWHM difference, the thickness and surface roughness of the epitaxial layer can also reflect the quality of the epitaxial layer, so the predicted data can also include the thickness, and/or surface roughness of the epitaxial layer. For instance, the predicted data can include the thickness, the surface roughness, and the FWHM difference.

For instance, assuming that the preset growth data are the O/Ga ratio is 300, the chamber pressure is 100 mbar, the temperature is 850 °C, the growth time is 3600 s, and the TMGa is 500 sccm. The preset growth data is input into the epitaxial layer prediction model, and the predicted data output by the epitaxial layer prediction model are the FWHM difference is 36 arcsec, the thickness is 7200nm, and the RMS is 12.3 nm.

In one embodiment, the training process of the epitaxial layer prediction model includes:
H10, acquiring a training dataset;
H20, inputting the training growth parameters in the training dataset into a preset machine learning model, and outputting the training predicted data of the beta-Ga₂O₃ epitaxial layer by the preset machine learning model;
H30, training the preset machine learning model based on the training predicted data and the label data to obtain the trained epitaxial layer prediction model.

The training dataset is pre-constructed and includes a plurality of training data groups, and each training data group includes training growth parameters and label data of a beta-Ga₂O₃ epitaxial layer. The training growth parameters include an O/Ga ratio, chamber pressure, temperature, and growth time. The label data of the beta-Ga₂O₃ epitaxial layer at least includes FWHM difference. In one embodiment, the label data of the beta-Ga₂O₃ epitaxial layer includes a thickness, a surface roughness and the FWHM difference of the beta-Ga₂O₃ epitaxial layer.

Further, the model structures of the preset machine learning model and the epitaxial layer prediction model are the same, and the difference between them is that the model parameters of the preset machine learning model are different from those of the epitaxial layer prediction model. The model parameters of the preset machine learning model are initial model parameters, and the model parameters of the epitaxial layer prediction model are model parameters trained by training datasets. Among them, the preset machine learning model can adopt a deep learning model, a polynomial regression model, or a large model. In one embodiment, the preset machine learning model adopts a polynomial regression model, and the polynomial regression model is trained by the training datasets to obtain the epitaxial layer prediction model.

In one embodiment, the step of training the preset machine learning model based on the training predicted data and the label data includes:
calculating a predicted difference value between the training predicted data and the label data;
when the predicted difference value is less than or equal to a preset difference threshold, determining a loss function term based on the training predicted data and the label data, and training the preset machine learning model based on the loss function term;
when the predicted difference value is greater than the preset difference threshold, determining the loss function term based on the training predicted data and the label data, setting a penalty term for the training predicted data, and training the preset machine learning model based on the loss function term and the penalty term.

The training predicted data is the predicted data of the epitaxial layer output by the preset machine learning model, and the label data is the truth data corresponding to the training growth parameters. Among them, the label data can be obtained by measuring the beta-Ga₂O₃ epitaxial layer, which is obtained by MOCVD epitaxy with training growth parameters. That is, after obtaining the training growth data, the beta-Ga₂O₃ epitaxial layer can be grown to obtain by MOCVD epitaxial process, and then measure the beta-Ga₂O₃ epitaxial layer to obtain the label data. Of course, the label data in each group of training data groups in the training datasets are the epitaxial data of the epitaxial layer whose epitaxial layer quality meets the preset requirements.

The predicted difference value is a difference between each epitaxial data item in the label data and the corresponding epitaxial data item in the training predicted data. For instance, the predicted difference value may include a difference value of the FWHM difference in the label data and the FWHM difference in the training predicted data, or one or more of difference value of the FWHM difference, and epitaxial layer thickness difference values and epitaxial layer surface roughness difference values. Of course, when the predicted difference value includes a plurality of difference values, the preset difference threshold includes a plurality of preset difference thresholds, and the plurality of preset difference thresholds correspond to the plurality of difference values included in the predicted difference value one by one. The predicted difference value is greater than the preset difference threshold means that at least one difference value of each difference value is greater than its corresponding difference threshold. The predicted difference value is less than or equal to the preset difference threshold means that all difference values are less than or equal to their corresponding difference thresholds.

In one embodiment, the predicted difference value includes the difference of the FWHM difference, and the preset difference threshold is the difference threshold of the FWHM difference. Correspondingly, the step of calculating a predicted difference value between the training predicted data and the label data includes: reading a predicted FWHM difference in the training predicted data and a labeled FWHM difference in the label data, and calculating a difference between the predicted FWHM difference and the labeled FWHM difference to obtain the predicted difference value. The predicted FWHM difference and the labeled FWHM difference are both used for reflecting a difference between the FWHM after epitaxy and the FWHM before epitaxy.

Further, when the predicted difference value is greater than the preset difference threshold, directly using the predicted difference value to construct a loss function may cause the preset machine learning model to be over-fitted, so that a penalty term can be added to the loss function when constructing the loss function based on the predicted difference value, and the preset machine learning model can be prevented from being over-fitted by the penalty term. The penalty term can be a regularization function, for instance, idge Regression, Least Absolute shrinkage, Selection Operator, and Elastic Net, etc. In addition, when the predicted difference value is less than or equal to the preset difference threshold, the loss function can be constructed directly based on the preset difference value. For instance, if the predicted FWHM difference corresponding to the training growth parameters is -20 arcsec and the labeled FWHM difference is +200 arcsec, then the predicted difference value is 220 which is greater than the preset difference threshold, and a penalty term is added to the loss function constructed based on the predicted FWHM difference. In addition, the loss function can be Determining Coefficient (R²), MSE, MAE, etc. In the embodiment of the present disclosure, the loss function adopts the determining coefficient.

In one embodiment, after the step of outputting predicted data of the beta-Ga₂O₃ epitaxial layer corresponding to the preset growth parameter by the epitaxial layer prediction model, the method further includes:
detecting whether the predicted data meets preset requirements;
when the predicted data does not meet the preset requirements, modifying the preset growth parameters to obtain modified growth parameters;
taking the modified growth parameters as the preset growth parameters, and re-executing the step of inputting the preset growth parameters into the trained epitaxial layer prediction model until the predicted data meets the preset requirements;
taking the preset growth parameters corresponding to the predicted data meeting the preset requirements as target growth parameters, and inputting the target growth parameters into MOCVD for beta-Ga₂O₃ epitaxy.

The preset requirements are preset, which are used to measure whether the preset growth data can be used as the basis of the actual growth data, for instance, the preset requirements are to reduce the FWHM difference by 40 arcsec, etc. When the predicted data meets the preset requirements, it means that the preset growth data can be used as the actual growth data, then the preset growth parameters can be used as the target growth parameters, and the target growth parameters can be input into the MOCVD process, and beta-Ga₂O₃ epitaxy is carried out in the MOCVD process. On the other hand, when the predicted data does not meet the preset requirements, the preset growth parameters can be modified to obtain modified growth parameters. When modifying the preset growth parameters, one parameter item or a plurality of parameter items in the preset growth parameters can be randomly adjusted, or the parameter items that need to be modified and their corresponding correction methods can be determined according to the preset experience table, and the parameter items can be modified according to the correction methods. Alternatively, the pre-trained modified network model inputs the predicted data and preset requirements into the modified network model, determines the parameter items to be modified and the modification method through the modified network model, and then modifies the parameter items to be modified. The modification method can increase or decrease the random data, or increase or decrease the set value.

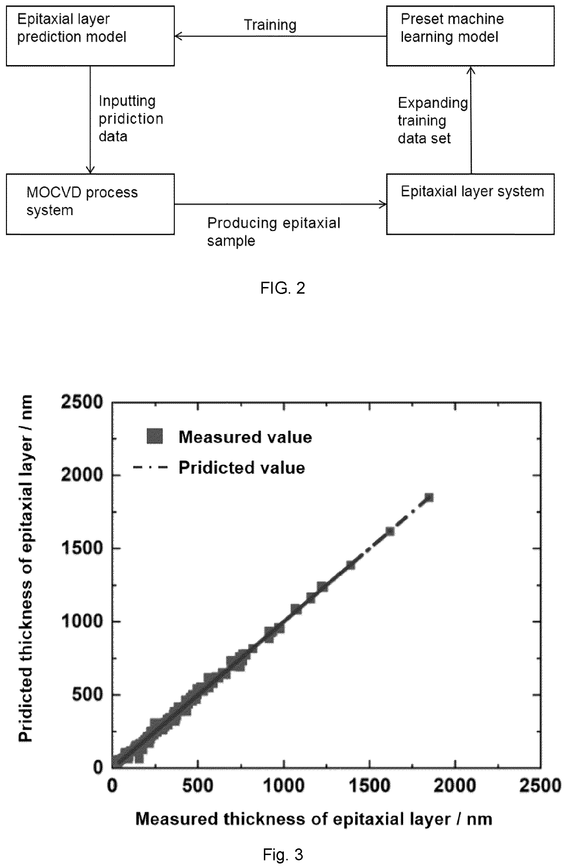

In one embodiment, as shown in Fig. 2, the method further includes:
generating a fine-tuning dataset based on the target growth parameters and corresponding epitaxial data;
fine-tuning the epitaxial layer prediction model based on the fine-tuning dataset.

After the target growth parameters are obtained, the target growth parameters are input into MOCVD for beta-Ga₂O₃ epitaxy to obtain beta-Ga₂O₃ epitaxial layer, and then the beta-Ga₂O₃ epitaxial layer is measured to obtain the label data corresponding to the target growth parameters, and the target growth parameters and the corresponding label data are used as a set of fine-tuning datasets, through which the epitaxial layer prediction model is fine-tuned to further improve the model accuracy of the epitaxial layer prediction model.

In summary, the present disclosure provides a prediction method for epitaxial layer of beta-Ga₂O₃ grown by MOCVD, including acquiring preset growth parameters, and inputting the preset growth parameters into a trained epitaxial layer prediction model, the preset growth parameters include an O/Ga ratio, chamber pressure, temperature, and growth time; outputting predicted data of the beta-Ga₂O₃ epitaxial layer corresponding to the preset growth parameter sby the epitaxial layer prediction model, the predicted data at least includes full width at half maximum. In the present disclosure, the machine learning model is used to predict the epitaxial layer, and the preset growth parameters are adjusted according to the predicted data to select the growth parameters suitable for the process window of beta-Ga₂O₃ epitaxial growth, and then the generated parameters are input into MOCVD for epitaxy. By combining machine learning with the MOCVD process, high-quality two-dimensional step-flow growth with TMGa and O₂ as reactants on unintentional oblique substrate is realized, and the quality of beta-Ga₂O₃ epitaxial layer grown by MOCVD is ensured.

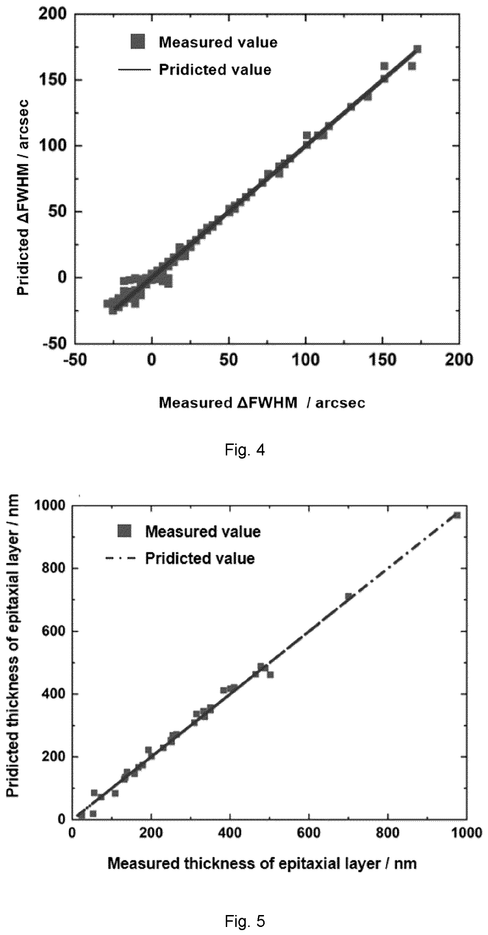

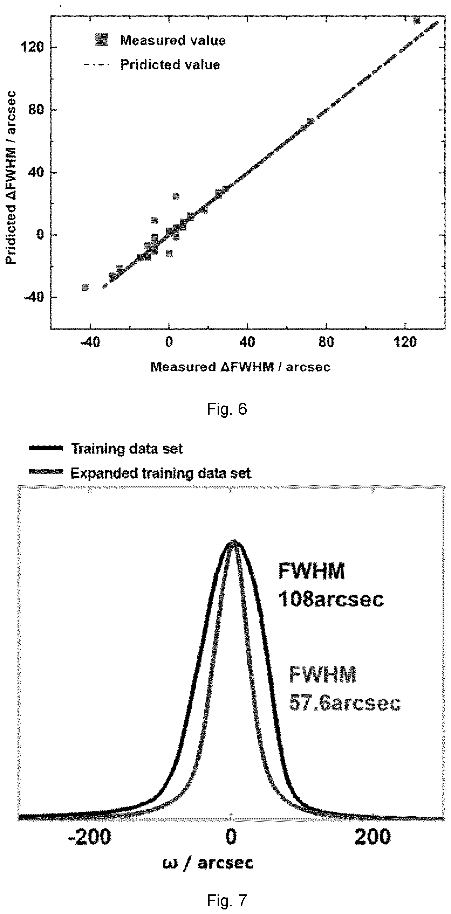

In order to further illustrate effects of the prediction method for epitaxial layer of beta-Ga₂O₃ grown by MOCVD, the performance of the epitaxial layer prediction model is tested, and the fitting result of the epitaxial layer prediction model on the training dataset is good. As shown in Figs. 3 and 4, the determination coefficient R² of epitaxial layer thickness is 0.996, and the determination coefficient R² of FWHM difference is 0.99. In the test set, as shown in Figs. 5 and 6, the determination coefficient R² of epitaxial layer thickness is 0.993, and the determination coefficient R² of FWHM difference is 0.962.

Furthermore, the model accuracy of the epitaxial layer prediction model can be further improved by expanding the training data with fine-tuning datasets and continuously fine-tuning the epitaxial layer prediction model. In the process of prediction by using the epitaxial layer prediction model, the target growth parameters with the FWHM difference reduced by nearly 40 arcsec can be obtained. According to the target growth parameters, beta-Ga₂O₃ epitaxy is carried out, and the obtained epitaxial layer is tested. As shown in Fig. 7, the FWHM of the epitaxial layer is actually reduced by 50 arcsec, and the surface of the tested sample is smooth and bright. AFM test shows that the surface roughness of the epitaxial layer is only 0.144 nm in the range of 1x1µm.

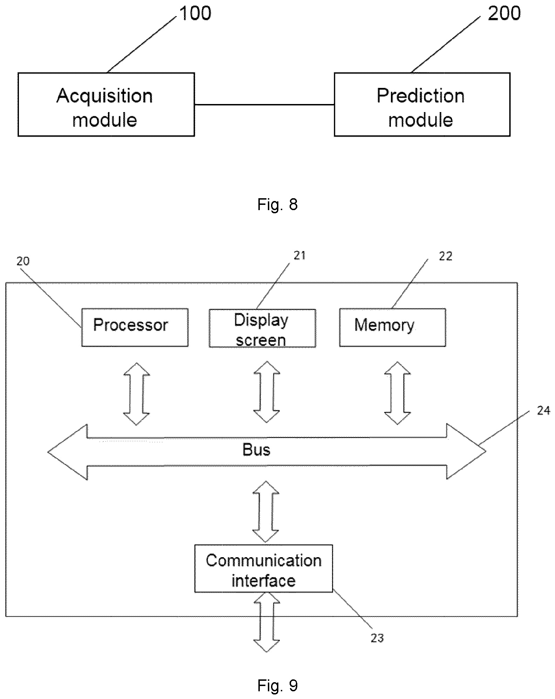

Based on the prediction method for epitaxial layer of beta-Ga₂O₃ grown by MOCVD, a prediction device for epitaxial layer of beta-Ga₂O₃ grown by MOCVD is provided. As shown in Fig. 8, the prediction device includes:
acquisition module 100, used for acquiring preset growth parameters, and inputting the preset growth parameters into a trained epitaxial layer prediction model, the preset growth parameters include an O/Ga ratio, chamber pressure, temperature, and growth time;
prediction module 200, used for outputting predicted data of the beta-Ga₂O₃ epitaxial layer corresponding to the preset growth parameters by the epitaxial layer prediction model, the predicted data at least includes FWHM difference.

Based on the prediction method for epitaxial layer of beta-Ga₂O₃ grown by MOCVD, a computer-readable storage medium is provided in the present disclosure. One or more programs are stored in the computer-readable storage medium, and the one or more programs can be executed by one or more processors to realize the steps in the prediction method for epitaxial layer of beta-Ga₂O₃ grown by MOCVD.

Based on the prediction method for epitaxial layer of beta-Ga₂O₃ grown by MOCVD, a terminal device is provided in the present disclosure. As shown in Fig. 9, the terminal device at least includes a processor 20, a display screen 21, and a memory 22, further includes a communication interface 23, and a bus 24. The processor 20, the display screen 21, the memory 22, and the communication interface 23 can communicate with each other by the bus 24. The display screen 21 is configured to display a user guidance interface preset in an initial setting mode. The communication interface 23 can transmit information. The processor 20 can call the logic instructions in the memory 22 to execute the method in the above embodiment.

In addition, the logic instructions in the memory 22 can be realized in the form of software functional units and can be stored in a computer-readable storage medium when they are sold or used as independent products.

As a computer-readable storage medium, the memory 22 is configured to store software programs and computer-executable programs, such as program instructions or modules corresponding to the methods in the embodiments of the present disclosure. The processor 20 executes functional applications and data processing by running software programs, instructions, or modules stored in the memory 22, that is, the method in the above embodiment is realized.

The memory 22 may include a storage program area and a storage data area, the storage program area may store an operating system and an application program required by at least one function; The storage data area can store data created according to the use of the terminal equipment, etc. In addition, the memory 22 may include a high-speed random access memory and may also include a non-transitory memory. For instance, U disk, mobile hard disk, Read-Only Memory (ROM), Random Access Memory (RAM), magnetic disk or optical disk, and other media that can store program codes can also be transient storage media.

In addition, the processes of loading and executing a plurality of instructions in the storage medium and the terminal device have been described in detail in the above method, and will not be described here one by one.

Finally, it should be noted that the above are only embodiments of the present disclosure. Those skilled in the art should understand that they may still modify the technical scheme described in the aforementioned embodiments or replace some of the technical features equally. These various modifications, supplements, or substitutions in a similar way, as long as they do not deviate from the methods of the present disclosure or exceed the scope defined by the present disclosure, should belong to the protection scope of the present disclosure.

## Claims

1. A prediction method for epitaxial layer of beta-Ga₂O₃ grown by MOCVD, comprising:
acquiring preset growth parameters, and inputting the preset growth parameters into a trained epitaxial layer prediction model, wherein the preset growth parameters comprises an O/Ga ratio, chamber pressure, temperature, and growth time;
outputting predicted data of the beta-Ga₂O₃ epitaxial layer corresponding to the preset growth parameters by the epitaxial layer prediction model, wherein the predicted data at least comprises full width at half maximum difference.

2. The prediction method for epitaxial layer of beta-Ga₂O₃ grown by MOCVD according to claim 1, wherein training process of the epitaxial layer prediction model comprises:
acquiring a training dataset, wherein the training dataset comprises a plurality of training data groups, and each training data group comprises training growth parameters and label data of a beta-Ga₂O₃ epitaxial layer;
inputting the training growth parameters in the training dataset into a preset machine learning model, and outputting the training predicted data of the beta-Ga₂O₃ epitaxial layer by the preset machine learning model;
training the preset machine learning model based on the training predicted data and the label data to obtain the trained epitaxial layer prediction model.

3. The prediction method for epitaxial layer of beta-Ga₂O₃ grown by MOCVD according to claim 2, wherein the step of training the preset machine learning model based on the training predicted data and the label data to obtain the trained epitaxial layer prediction model comprises:
calculating a predicted difference value between the training predicted data and the label data;
when the predicted difference value is less than or equal to a preset difference threshold, determining a loss function term based on the training predicted data and the label data, and training the preset machine learning model based on the loss function term;
when the predicted difference value is greater than the preset difference threshold, determining the loss function term based on the training predicted data and the label data, setting a penalty term for the training predicted data, and training the preset machine learning model based on the loss function term and the penalty term.

4. The prediction method for epitaxial layer of beta-Ga₂O₃ grown by MOCVD according to claim 3, wherein both of the training predicted data and the label data comprise the full width at half maximum difference; the step of calculating a predicted difference value between the training predicted data and the label data comprises:
reading a predicted full width at half maximum difference in the training predicted data and a labeled full width at half maximum difference in the label data, wherein the predicted full width at half maximum difference and the labeled full width at half maximum difference are both configured to reflect a difference between the full width at half maximum after epitaxy and the full width at half maximum before epitaxy;
calculating a difference between the predicted full width at half maximum difference and the labeled full width at half maximum difference to obtain the predicted difference value.

5. The prediction method for epitaxial layer of beta-Ga₂O₃ grown by MOCVD according to claim 1, wherein the predicted data further comprises thickness and/or surface roughness.

6. The prediction method for epitaxial layer of beta-Ga₂O₃ grown by MOCVD according to claim 1, after the step of outputting predicted data of the beta-Ga₂O₃ epitaxial layer corresponding to the preset growth parameters by the epitaxial layer prediction model, the method further comprises:
detecting whether the predicted data meets preset requirements;
when the predicted data does not meet the preset requirements, modifying the preset growth parameters to obtain modified growth parameters;
taking the modified growth parameters as the preset growth parameters, and re-executing the step of inputting the preset growth parameters into the trained epitaxial layer prediction model until the predicted data meets the preset requirements;
taking the preset growth parameters corresponding to the predicted data meeting the preset requirements as target growth parameters, and inputting the target growth parameters into the MOCVD for epitaxial growth of beta-Ga₂O₃.

7. The prediction method for epitaxial layer of beta-Ga₂O₃ grown by MOCVD according to claim 6, the method further comprises:
generating a fine-tuning dataset based on the target growth parameters and corresponding epitaxial data;
fine-tuning the epitaxial layer prediction model based on the fine-tuning dataset.

8. A prediction device for epitaxial layer of beta-Ga₂O₃ grown by MOCVD, comprising:
an acquisition module, configured to acquire preset growth parameters, and inputting the preset growth parameters into a trained epitaxial layer prediction model, wherein the preset growth parameters comprise an O/Ga ratio, chamber pressure, temperature, and growth time;
a prediction module, configured to output predicted data of the beta-Ga₂O₃ epitaxial layer corresponding to the preset growth parameters by the epitaxial layer prediction model, wherein the predicted data at least comprises full width at half maximum difference.

9. A computer-readable storage medium, wherein one or more programs are stored in the computer-readable storage medium, and the one or more programs can be executed by one or more processors to realize the steps in the prediction method for epitaxial layer of beta-Ga₂O₃ grown by MOCVD according to any one of claims 1-7.

10. A terminal device, comprising a processor and a memory;
a computer-readable program executable by the processor is stored in the memory;
when the processor executes the computer-readable program, the processor realizes the steps in the prediction method for epitaxial layer of beta-Ga₂O₃ grown by MOCVD according to any one of claims 1-7.
